# EUROPEAN PATENT APPLICATION

(11) **EP 2 261 318 A1**
(43) Date of publication of application: **15.12.2010**
(21) Application number: 09723651.7
(22) Date of filing: 27.02.2009
(51) Int. Cl.: C12N 1/14, C12P 19/02, C12N 9/26, C12N 9/42

(54) **FILAMENTOUS FUNGUS CULTURE MIXTURE HAVING CELLULOSE-DECOMPOSING ACTIVITY OR DRIED PRODUCT THEREOF, AND METHOD FOR PRODUCTION OF GLUCOSE USING THE MIXTURE OR THE DRIED PRODUCT**

(30) Priority: 28.03.2008 JP 2008086792
(71) Applicant: Asahi Breweries, Ltd., Sumida-ku Tokyo 130-8602 (JP)
(72) Inventor: FUKUDA, Kazuro, Moriya-shi Ibaraki 302-0106 (JP)
(74) Representative: Müller, Christian Stefan Gerd
(86) International application number: PCT/JP2009/053748
(87) International publication number: WO 2009/119252

(57) **Abstract**

An objective of the present invention is to provide a method for enzymatically saccharifying a cellulosic substance in an extremely efficient, simple, and inexpensive manner. A solution to the object is a mixture or a dried product thereof exhibiting cellulolytic activity containing a culture liquid or a supernatant liquid of a genus Trichoderma filamentous fungus and a culture product of Aspergillus kawachii.

## Description

### TECHNICAL FIELD

The present invention relates to a method for saccharifying cellulose, particularly to a method for saccharifying cellulose using a mixture of culture products of filamentous fungi which exhibits cellulolytic activity.

### BACKGROUND ART

Recently, methods for efficiently saccharifying cellulose have been searched in order to utilize cellulose resources effectively. In the natural world, cellulose is degraded mainly by microorganisms, and it is known that various microorganisms such as bacteria and filamentous fungi produce cellulolytic enzymes.

These microorganisms secrete a plurality of cellulolytic enzymes outside the fungus bodies, and cellulose will be degraded to glucose mainly via cello-oligosaccharide and cellobiose through cooperative action of various cellulolytic enzymes having different mechanisms of action. Cellulolytic enzymes are generally called cellulase as a collective term for the enzymes which can hydrolyze cellulose.

The genus Trichoderma is a most promising enzyme source of cellulase, and is widely used worldwide. Meanwhile, Aspergillus kawachii, which is a white koji mold, is a koji mold unique to Japan used in making shochu. Aspergillus kawachii is widely utilized for saccharification of cereal raw materials. However, Aspergillus kawachii does not have particularly excellent cellulase-secreting ability, and thus it has been rarely used for saccharification of cellulose.

Non-Patent Document 1 describes that, as a result of degradation of cellulose using a culture supernatant liquid of Aspergillus aculeatus in combination with a commercial cellulase enzyme, synergistic effect was identified in degradation efficiency.

Non-Patent Document 2 describes that, by using the cellulase derived from genus Trichoderma fungus bodies in combination with the cellulase derived from Aspergillus aculeatus, synergistic action was identified in ability of purifying monosaccharide from cellulose.

However, Aspergillus aculeatus is different in nature from Aspergillus kawachii in that it intrinsically has high ability of producing and secreting cellulase. In these conventional arts, enzyme agents are used, resulting in high cost and cumbersome operations. Moreover, reported degradation and saccharification efficiencies are considerably lower than the level at which profits can be expected on large-scale operations, therefore, when practical applications are intended, reported efficiencies are still insufficient.
[Non-Patent Document 1] J. Ferment. Technol., Vol. 57, pp. 151-159, 1979
[Non-Patent Document 2] Hakkokogaku, Vol. 60, pp. 333-341, 1982

### DISCLOSURE OF THE INVENTION

### PROBLEMS TO BE SOLVED BY THE INVENTION

The present invention is intended to solve the above-described conventional problems, and an objective of the present invention is to provide a method for enzymatically saccharifying a cellulosic substance in an extremely efficient, simple, and inexpensive manner.

### MEANS FOR SOLVING THE PROBLEMS

The present invention provides a mixture of a culture liquid or a supernatant liquid of a genus Trichoderma filamentous fungus and a culture product of Aspergillus kawachii, or a dried product thereof, which exhibits cellulolytic activity, whereby the above-described objective is accomplished.

In an embodiment, the culture product of Aspergillus kawachii is a culture liquid or a supernatant liquid.

In an embodiment, the culture product of Aspergillus kawachii is a solid culture product or an extract liquid thereof.

In an embodiment, cellulase activity of the culture liquid or the supernatant liquid of the genus Trichoderma filamentous fungus is not less than 50 U/ml, and hemicellulase activity of the culture liquid, the supernatant liquid, or the solid culture product extract liquid of Aspergillus kawachii is not less than 50 U/ml.

In an embodiment, mixing ratio of the culture liquid or the supernatant liquid of the genus Trichoderma filamentous fungus to the culture liquid, the supernatant liquid, or the solid culture product extract liquid of Aspergillus kawachii is within a range of 10 : 90 to 90 : 10 in volume ratio.

In an embodiment, the genus Trichoderma filamentous fungus is Trichoderma reesei or Trichoderma viride.

In an embodiment, the genus Trichoderma filamentous fungus is Trichoderma reesei.

The present invention also provides a method for producing glucose including a step of saccharifying a cellulose raw material using the mixture or the dried product thereof.

In an embodiment, the saccharification is performed under conditions of 30 to 70°C and pH 3 to 7.

### EFFECT OF THE INVENTION

The mixture of culture products of filamentous fungi according to the present invention has remarkably high cellulose degradation and saccharification ability. Thus, it can be used as it is for saccharification of cellulose, and so there is no need to use enzyme agents. As a result, cellulose can be inexpensively saccharified by simple operations.

### BEST EMBODIMENT FOR CARRYING OUT THE INVENTION

A genus Trichoderma filamentous fungus is known as an enzyme source for cellulase necessary for saccharification of cellulose. A genus Trichoderma filamentous fungus to be used in the present invention is not particularly limited as long as it can produce cellulase. A preferred genus Trichoderma filamentous fungus is Trichoderma reesei or Trichoderma viride. Particularly preferably, it is Trichoderma reesei.

Synergistic effect is identified in cellulolytic activity when a culture liquid or a supernatant liquid of the genus Trichoderma filamentous fungus is used in combination with a culture product of Aspergillus kawachii. Cellulose degradation and saccharification ability of a culture liquid or a supernatant liquid of filamentous fungi can be remarkably enhanced by utilizing the synergistic effect.

Mycological properties of filamentous fungi Trichoderma reesei and Trichoderma viride are described in, for example, E.G. Simmons, Abst. 2nd International Mycological Congress, Tampa, FL, U.S.A, August 1977, p. 618.

The culture liquid or the supernatant liquid of the genus Trichoderma filamentous fungus can be prepared by a method conventionally performed. That is, spores, hyphae, or pre-culture liquids obtained by culturing in advance, of the fungal strains, are inoculated into a liquid medium and cultured. For example, when spores are cultured, it is preferable to culture the parent strains on potato dextrose agar media so as to allow the spores to be fully formed in advance. In the above-described case, the culture temperature is 20 to 33°C, and the culture period is 4 to 10 days.

As a carbon source of the medium, powdered cellulose, cellobiose, filter paper, general paper materials, sawdust, bran, chaff, bagasse, soybean meal, coffee grounds, starch, lactose, and the like are used. As a nitrogen source, inorganic ammonium salts such as ammonium sulfate and ammonium nitrate, and organic nitrogen-containing substances such as urea, amino acid, a meat extract, a yeast extract, polypeptone, and a protein degradation product are used. As an inorganic salt, KH₂PO₄, MgSO₄-7H₂O, CaCl₂·2H₂O, Fe₂Cl₃·6H₂O, MnCl₂·4H₂O, ZnSO₄·7H₂O, and the like are used. If needed, a medium containing organic micronutrients is used.

Liquid culture is performed, using the above-described medium, at a culture temperature of 20 to 33°C, preferably 28 to 30°C, and at a culture pH of 4 to 6, for 4 to 10 days, with normal aeration-stirring type culture apparatuses. As described above, the culture liquid of the genus Trichoderma filamentous fungus is obtained. Then, the culture supernatant liquid of the genus Trichoderma filamentous fungus is obtained by removing the fungus body from this culture liquid by a publicly known method such as centrifugation or filtration.

Examples of the enzymes contained in the culture liquid or the culture supernatant liquid of the genus Trichoderma filamentous fungus include cellulase, hemicellulase, and mannanase.

The cellulase activity of the culture liquid or the culture supernatant liquid thus obtained is not less than 10 U/ml, preferably not less than 50 U/ml, for example, 50 to 150 U/ml, 80 to 120 U/ml, more preferably approximately 100 U/ml. When the cellulase activity of the culture liquid or the culture supernatant liquid is less than 10 U/ml, time required for degradation of cellulose will be prolonged.

It is to be noted that the above-described cellulase activity can be obtained by quantitating the amount of reducing sugar produced by hydrolysis of a substrate, which is carboxymethyl cellulose (CMC), by a dinitrosalicylic acid (DNS) method.

More specifically, to 1 ml of a 1% CMC substrate solution (prepared by dissolving "low viscosity" manufactured by Sigma-Aldrich, Inc. in a 100 mM acetate buffer (pH 5)), 1 ml of the culture liquid or the culture supernatant liquid is added, and an enzymatic reaction is allowed to proceed at 40°C for exactly 10 minutes. Subsequently, to the resulting mixture, 4 ml of a DNS reagent (containing 0.75% dinitrosalicylic acid, 1.2% sodium hydroxide, 22.5% potassium sodium tartrate tetrahydrate, and 0.3% lactose monohydrate) is added and the mixture is thoroughly mixed to terminate the reaction. In order to quantitate the amount of reducing sugar contained in this reaction-terminated liquid, the reaction-terminated liquid is heated exactly for 15 minutes in a boiling water bath. Subsequently, the mixture is cooled to room temperature, and then the amount of reducing sugar, which corresponds to glucose, is quantitated by measuring absorbance at 540 nm. One unit of cellulase activity is expressed as the amount of enzyme capable of producing reducing sugar in an amount equivalent to 1 µmol of glucose per minute.

Aspergillus kawachii is also called koji mold. It is a kind of fungi that has been utilized for brewing and production of fermented food products since ancient times, and thus is a safe microorganism. Microorganisms belonging to the genus Aspergillus are known to produce α-amylase, and utilized for saccharification of starch.

Mycological properties of a filamentous fungus Aspergillus kawachii is described in, for example, Koji-gaku, written and edited by Hideya MURAKAMI, March 25, 1987, p. 78.

The culture product of Aspergillus kawachii may be prepared by a method conventionally performed. For example, the culture product of Aspergillus kawachii can be a solid culture product obtained by solid culture, or can be a culture liquid or a supernatant liquid obtained by liquid culture. Also, the culture product of Aspergillus kawachii can be a solid culture product extract liquid obtained by immersing the solid culture product in water, brine, or the like to extract its components, and then removing insoluble components such as the fungus body by filtration or the like.

It is to be noted that, taking into consideration the convenience in industrial-scale handling, the culture product of Aspergillus kawachii is preferably a culture liquid or a supernatant liquid.

When performing liquid culture of Aspergillus kawachii, spores, hyphae, or pre-culture liquids obtained by culturing in advance, of fungal strains, are inoculated into a liquid medium and cultured. For example, when spores are cultured, it is preferable to culture the parent strains on potato dextrose agar media so as to allow the spores to be fully formed in advance. In the above-described case, the culture temperature is 30 to 40°C, and the culture period is 3 to 7 days.

A usable carbon source is preferably maltose or starch, and an appropriate concentration thereof is approximately 2%. As a nitrogen source, peptone is preferable, and an appropriate concentration thereof is approximately 0.5%. Besides, a yeast extract, corn steep liquor, monopotassium phosphate, magnesium sulfate, and the like are required. Although the above-described carbon source is preferably maltose or starch, other than the above, any of the culture media normally used for fungi is applicable.

Liquid culture is performed, using the above-described medium, at a culture temperature of 30 to 40°C, preferably 35 to 38°C, and at a culture pH of 4 to 6, for 2 to 4 days, with normal aeration-stirring type culture apparatuses. As described above, the culture liquid of Aspergillus kawachii can be obtained. Then, the culture supernatant liquid of Aspergillus kawachii is obtained by removing the fungus body from the culture liquid by a publicly known method such as centrifugation or filtration.

When performing solid culture of Aspergillus kawachii, spores or hyphae of fungal strains are inoculated onto a solid medium and cultured. For example, when spores are cultured, it is preferable to culture the parent strains on potato dextrose agar media so as to allow spores to be fully formed in advance. In the above-described case, a culture temperature is 30 to 40°C, and a culture period is 3 to 7 days.

A usable solid medium, besides bran, is preferably a starch raw material, which are cereals, such as rice, barley, foxtail millet and Japanese barnyard millet, and beans, and the like. Particularly preferably, bran is used.

For solid culture, similarly to a method for producing koji for shochu, a method employing a koji tray or the like during production of koji and a mechanical koji-producing device are used. The above-described medium is steamed, into which a fungal strain is inoculated, and it is cultured at a culture temperature of 30 to 40°C, preferably 35 to 38°C, at a culture pH of 4 to 6, for 2 to 4 days. As described above, the solid culture product of Aspergillus kawachii can be obtained. Subsequently, this culture product is immersed in water to extract its components, and the fungus body is removed by filtration, whereby a solid culture filtrate liquid of Aspergillus kawachii is obtained.

Examples of the enzymes contained in the culture liquid, the culture supernatant liquid, the solid culture product or the extract liquid thereof of Aspergillus kawachii include cellulase, hemicellulase, mannanase, pectinase, and protease.

The hemicellulase activity of the culture liquid, the culture supernatant liquid, or the solid culture product extract liquid thus obtained is not less than 10 U/ml, preferably not less than 50 U/ml, for example, 50 to 150 U/ml, 80 to 120 U/ml, more preferably approximately 100 U/ml. When the hemicellulase activity of the culture liquid, the culture supernatant liquid, or the solid culture product extract liquid is less than 10 U/ml, time required for degradation of cellulose will be prolonged.

It is to be noted that the above-described hemicellulase activity can be obtained by quantitating the amount of reducing sugar produced by enzymatic hydrolysis of a substrate, which is xylan derived from oat spelts, with DNS, and observing an increase in the absorbance at 540 nm.

More specifically, to 1.9 ml of a 1% xylan substrate solution (prepared by dissolving "xylan, from oat spelts" manufactured by Sigma-Aldrich, Inc. in a 200 mM acetate buffer (pH 4.5)), 0.1 ml of the culture liquid or the culture supernatant liquid is added, and an enzymatic reaction is allowed to proceed at 40°C for exactly 10 minutes. Subsequently, to the resulting mixture, 4 ml of a DNS reagent (containing 0.75% dinitrosalicylic acid, 1.2% sodium hydroxide, 22.5% potassium sodium tartrate tetrahydrate, and 0.3% lactose monohydrate) is added and the mixture is thoroughly mixed to terminate the reaction. In order to quantitate the amount of reducing sugar contained in this reaction-terminated liquid, the reaction-terminated liquid is heated exactly for 15 minutes in a boiling water bath. Subsequently, the mixture is cooled to room temperature, and then the amount of reducing sugar, which corresponds to xylose, is quantitated by measuring absorbance at 540 nm. One unit of hemicellulase activity is expressed as the amount of enzyme capable of producing reducing sugar in an amount equivalent to 1 µmol of xylose per minute under conditions of 40°C for 10 minutes.

The mixture which exhibits cellulolytic activity of the present invention is obtained by mixing the culture liquid or the culture supernatant liquid of the genus Trichoderma filamentous fungus and the culture product of Aspergillus kawachii. Both the culture liquid and the culture supernatant liquid can be similarly used in preparation of the mixture of the present invention. It is to be noted that preparation of the culture liquid does not require operations for removing the fungus body; thus, its preparation method is simple. Meanwhile, there is substantially no fungus body in the culture supernatant liquid; thus, the supernatant liquid is excellent in storage stability.

No particular limitation is imposed on a method for mixing the culture liquid or the supernatant liquid of the genus Trichoderma filamentous fungus and the culture product of Aspergillus kawachii. For example, when the culture product of Aspergillus kawachii is a solid culture product, the solid culture product can be added, as it is, to the culture liquid of the genus Trichoderma filamentous fungus or the like and mixed. Alternatively, the solid culture product extract liquid can be mixed with the culture liquid of the genus Trichoderma filamentous fungus or the like. Also, for example, when the culture product of Aspergillus kawachii is a liquid culture product or a supernatant liquid, the culture product of Aspergillus kawachii and the culture liquid or the supernatant liquid of the genus Trichoderma filamentous fungus can be directly mixed. The culture liquid of the genus Trichoderma filamentous fungus and the culture product of Aspergillus kawachii can be each filtrated before mixing, or can be filtrated after mixing.

The mixing ratio of the culture liquid or the supernatant liquid of the genus Trichoderma filamentous fungus to the culture liquid, the supernatant liquid, or the solid culture product extract liquid of Aspergillus kawachii is, for example, 10 : 90 to 90 : 10, preferably 20 : 80 to 80 : 20, more preferably 30 : 70 to 70 : 30, 40: 60 to 60 : 40, even more preferably 50 : 50, in volume ratio. When the mixing ratio is less than 10 : 90 or more than 90 : 10, the saccharification efficiency of cellulose will be reduced. Also, the mixture thus obtained can also be provided as a dried product, which can be prepared by a method normally employed such as freeze-drying.

A cellulose raw material can be degraded and saccharified using the above-described mixture. Cellulose refers to a molecular chain in which glucose is polymerized by β-1,4-glucoside linkage, or a derivative thereof (for example, ones subjected to derivatization such as carboxymethylation, aldehyde-modification, or esterification, such as carboxymethyl cellulose), or a substance in which a plurality of the above-described molecular chains or derivatives thereof are bonded together (such as a cellulose fiber).

The cellulose raw material is preferably a water-insoluble fibrous substance containing cellulose. The cellulose raw material may be derived from either a plant or an animal, and examples of plants and animals producing the cellulose raw material include bagasse, beer residue, wood, bamboo, wheat straw, rice straw, corn cobs, cotton, ramie, kenaf, beet, hoya, and bacterial cellulose, and the like. Also, in the present invention, in addition to natural cellulose substances produced by the above-described plants and animals, regenerated cellulose substances obtained by once chemically or physically dissolving or swelling natural cellulose substances and then regenerating them, and cellulose derivative substances obtained by chemically modifying cellulose raw materials can be used.

The above-described cellulose substance may be, for industrial use, any of natural cellulose raw materials such as pulp, powdered cellulose, and crystalline cellulose, regenerated cellulose such as rayon, various regenerated cellulose raw materials such as alkali cellulose and phosphoric acid swollen cellulose, and various kinds of cellulose derivative materials such as sodium carboxymethyl cellulose. However, in order to use glucose to be obtained for pharmaceutical products, foodstuffs, and cosmetics, it is more preferable to use natural cellulose raw materials. As a raw material, among the above-described cellulose raw materials, one kind thereof or a mixture of two or more kinds thereof can be used.

A publicly known method may be used as a method for saccharifying cellulose. Although no particular limitation is imposed on the method, one example thereof is a method including suspending a cellulose raw material as a substrate in an aqueous vehicle; adding the above-described mixture thereto; and heating the resulting mixture while stirring or shaking to allow a saccharification reaction to proceed. Instead of the above-described mixture exhibiting cellulolytic activity, a dried product thereof or a solution containing the dried product dispersed or dissolved therein may be used.

The cellulose raw material is preferably subjected to delignification in advance. Reaction conditions such as a method of suspension, a method of stirring, a method of adding the above-described mixture, an order of addition, and a concentration at which the above-described operations are carried out are appropriately adjusted so as to obtain glucose at higher yield.

In saccharification of cellulose, pH and temperature of the reaction liquid may be set within such a range that enzymes will not be inactivated. Generally, when performing a reaction under normal pressure, temperature may be within a range of 30 to 70°C, and pH may be within a range of 3 to 7. Also, similarly to the above, the pressure, temperature, and pH are also appropriately adjusted so as to obtain glucose at higher yield. Under normal pressure in an acetate or phosphate buffer, the saccharification is preferably performed at a temperature of 50 to 60°C and a pH of 4 to 6. Reaction time is generally 6 to 147 hours, preferably 24 to 72 hours.

An aqueous solution containing glucose is obtained by saccharifying glucose. The aqueous solution thus obtained can be subjected to a purification treatment such as decolorization, desalination, and enzyme removal, as needed. Although no particular limitation is imposed on the method of purification as long as it is a publicly known method, for example, a filtration treatment such as an activated carbon treatment, an ion exchange resin treatment, a chromatography treatment, microfiltration, ultrafiltration, or reverse osmosis filtration, and a crystallization treatment may be used. The above-described treatment can be used singly or two more kinds thereof may be used in combination.

The aqueous solution mainly composed of glucose purified by the above-described method can be used as it is, or solidified by drying as needed. Although no particular limitation is imposed on the method of drying as long as it is a publicly known method, for example, spray-drying, freeze-drying, drum drying, thin film drying, tray drying, flash drying, and vacuum drying can be used. The above-described method can be used singly or two more kinds thereof can be used in combination.

### EXAMPLES

The present invention will be more specifically described with reference to the following examples; however, the present invention will not be limited to these examples.

### Example 1

Trichoderma reesei QM9414 (NBRC 31329) was cultured on a potato dextrose agar medium at 28°C for 7 days so as to allow spores to be fully formed. One platinum loop thereof was inoculated into a 500 ml-Erlenmeyer flask with baffles containing crystalline cellulose (manufactured by Asahikasei Chemicals Corporation, trade name: Ceolus): 1 g, KH₂PO₄: 0.2 g, (NH₄)₂SO₄: 0.14 g, CaCl₂·2H₂O: 0.03 g, MgSO₄·7H₂O: 0.03 g, polypeptone: 0.2 g, a yeast extract: 0.05 g, Tween 80: 0.1 ml, a trace element liquid (a liquid obtained by dissolving and suspending 6 mg of H₃BO₄, 26 mg of (NH₄)6Mo₇O₂₄·4H₂O, 100 mg of FeCl₃·6H₂O, 40 mg of CuSO₄·5H₂O, 8 mg of MnCl₂·4H₂O, and 200 mg of ZnSO₄·7H₂O in 100 ml of water): 0.1 ml and water: 100 ml, followed by shaking culture at 28°C for 7 days. On day 7, the culture liquid was filtrated to obtain a supernatant liquid.

The cellulase activity of the supernatant liquid thus obtained was 50 U/ml as measured by the above-described method.

Meanwhile, Aspergillus kawachii (NBRC4308) was cultured on a potato dextrose agar medium at 37°C for 4 days so as to allow spores to be fully formed. One platinum loop thereof was inoculated into a 500 ml-Erlenmeyer flask with baffles containing wheat bran: 3 g, K₂HPO₄: 0.5 g, and water: 100 ml, followed by shaking culture at 37°C for 3 days. On day 3, the culture liquid was filtrated to obtain a supernatant liquid.

The hemicellulase activity of the supernatant liquid thus obtained was 50 U/ml as measured by the above-described method.

The cellulose raw material to be subjected to saccharification was subjected to delignification in accordance with the following method. Beer residue, rice straw, wheat straw, and bagasse were each finely pulverized and suspended in 0.3 N NaOH, and treated at 120°C for 15 minutes. Subsequently, the product thus obtained was thoroughly washed with water and dried. Powdered cellulose (manufactured by Nippon Paper Chemicals Co., Ltd., trade name: KC flock W-50) was subjected to saccharification as it was.

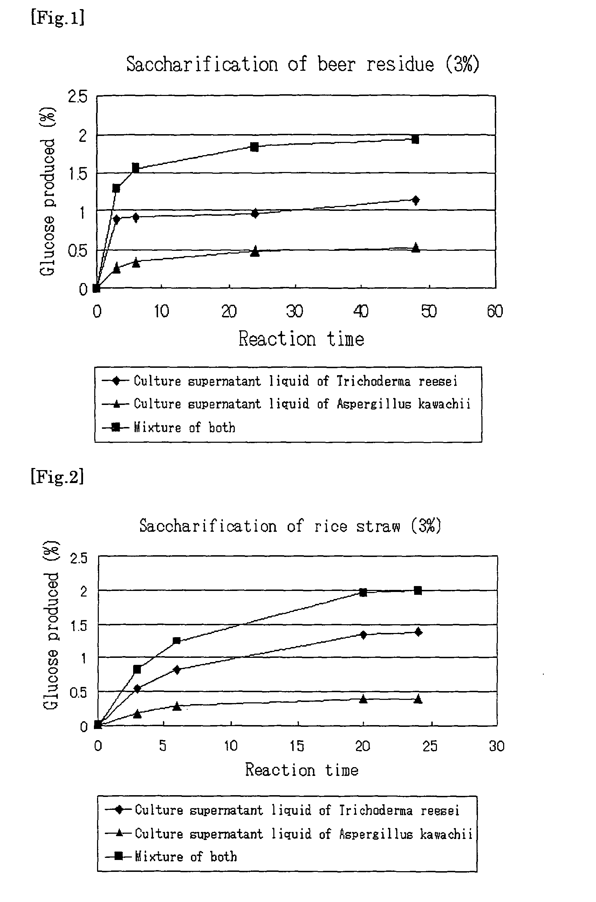

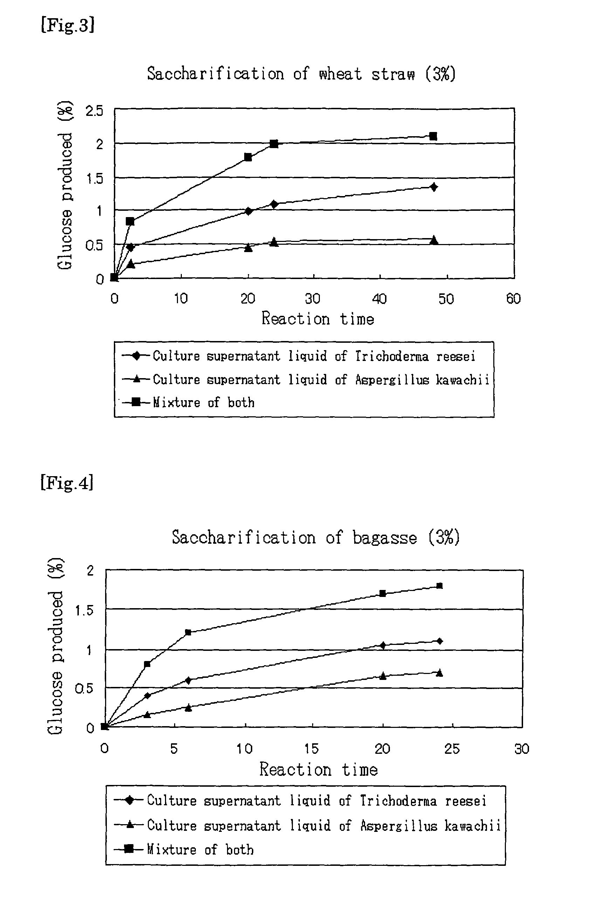

The cellulose raw material was saccharified by shaking a liquid (a 3% cellulose raw material liquid) consisting of the cellulose raw material: 0.3 g, the culture supernatant liquid of Trichoderma reesei: 4.75 ml, the culture supernatant liquid of Aspergillus kawachii: 4.75 ml, and a 1 M acetate buffer (pH 4.8): 0.2 ml at 50°C at pH 4.8 for 24 to 48 hours. Then, glucose thus produced was measured by glucose CII-test Wako (Wako Pure Chemicals Industries, Ltd.). As a control, a culture supernatant liquid of Trichoderma reesei: 9.5 ml and a culture supernatant liquid of Aspergillus kawachii: 9.5 ml were each singly used instead of the mixture of the culture supernatant liquids. The results thus obtained are each shown in Figs. 1 to 5.

### Example 2

With altering the mixing ratio of the culture supernatant liquid of Trichoderma reesei and the culture supernatant liquid of Aspergillus kawachii, saccharification efficiencies were compared. For these culture supernatant liquids, ones prepared in a similar manner to Example 1 were used.

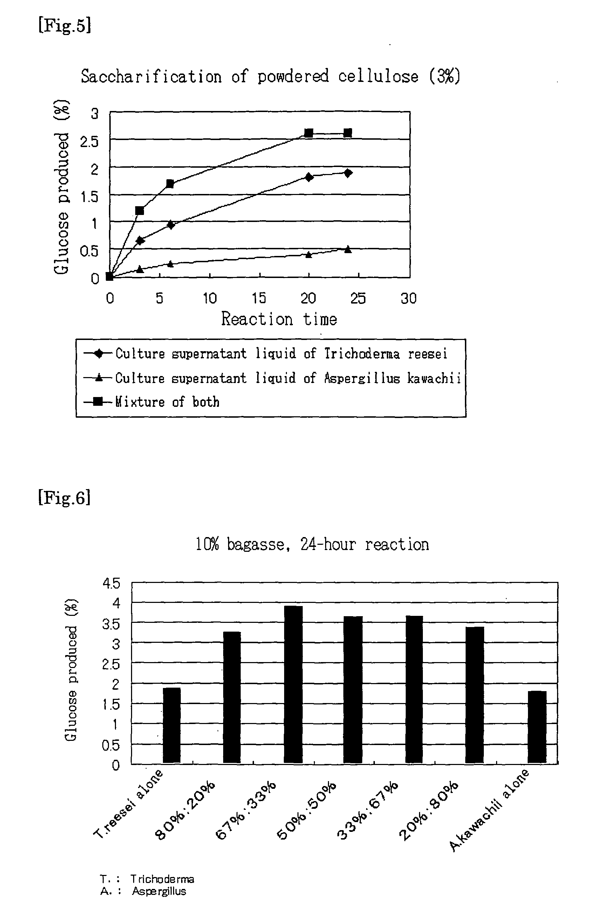

Saccharification was carried out by shaking a liquid (a 10% bagasse liquid) consisting of bagasse: 1 g, a mixture of the culture supernatant liquids: 8.8 ml, and a 1 M acetate buffer (pH 4.8): 0.2 ml at 50°C at pH 4.8 for 24 hours. Then, glucose thus produced was measured. The results thus obtained are shown in Fig. 6. The mixing ratio shown in the horizontal axis of the graph is the volume ratio.

### Example 3

A culture liquid of Trichoderma reesei and a culture liquid of Aspergillus kawachii were obtained in a similar manner to Example 1 except for omitting filtration. Subsequently, various kinds of mixtures having altered mixing ratios of the culture liquids were prepared and saccharification efficiencies were compared.

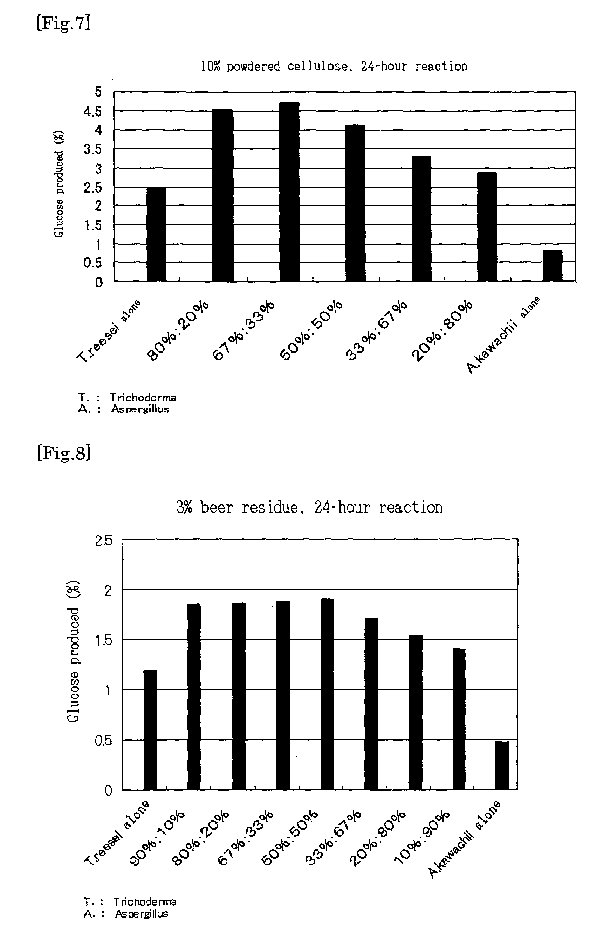

Saccharification was carried out by shaking a liquid (a 10% powdered cellulose liquid) consisting of powdered cellulose (trade name: KC flock): 1 g, a mixture of the culture liquids: 8.8 ml, and a 1 M acetate buffer (pH 4.8): 0.2 ml at 50°C at pH 4.8 for 24 hours. Then, glucose thus produced was measured. The results thus obtained are shown in Fig. 7. The mixing ratio shown in the horizontal axis of the graph is the volume ratio.

### Example 4

With altering the mixing ratio of the culture supernatant liquid of Trichoderma reesei and the culture supernatant liquid of Aspergillus kawachii, saccharification efficiencies were compared. For these culture supernatant liquids, ones prepared in a similar manner to Example 1 were used.

Saccharification was carried out by shaking a liquid (a 3% beer residue liquid) consisting of beer residue: 0.3 g, a mixture of the culture supernatant liquids: 9.5 ml, and a 1 M acetate buffer (pH 4.8): 0.2 ml at 50°C at pH 4.8 for 24 hours. Then, glucose thus produced was measured. The results thus obtained are shown in Fig. 8. The mixing ratio shown in the horizontal axis of the graph is the volume ratio.

### Example 5

With employing various kinds of culture supernatant liquids of the genus Aspergillus filamentous fungi in combination with a culture supernatant liquid of Trichoderma reesei, the synergistic effects on the saccharification efficiency were compared. For the culture supernatant liquid of Trichoderma reesei, one prepared in a similar manner to Example 1 was used.

Aspergillus awamori, NBRC4388, Aspergillus niger, NBRC31125, Aspergillus saitoi, ATCC 11363, Aspergillus oryzae, RIB40, and Aspergillus aculeatus, NBRC5330, were each cultured on potato dextrose agar media at 37°C for 4 days so as to allow spores to be fully formed. One platinum loop of each of them was inoculated into a 500 ml-Erlenmeyer flask with baffles containing wheat bran: 3 g, E₂HPO₄: 0.5 g, and water: 100 ml, followed by shaking culture at 37°C for 3 days. On day 3, each of the culture liquids was filtrated to obtain a supernatant liquid.

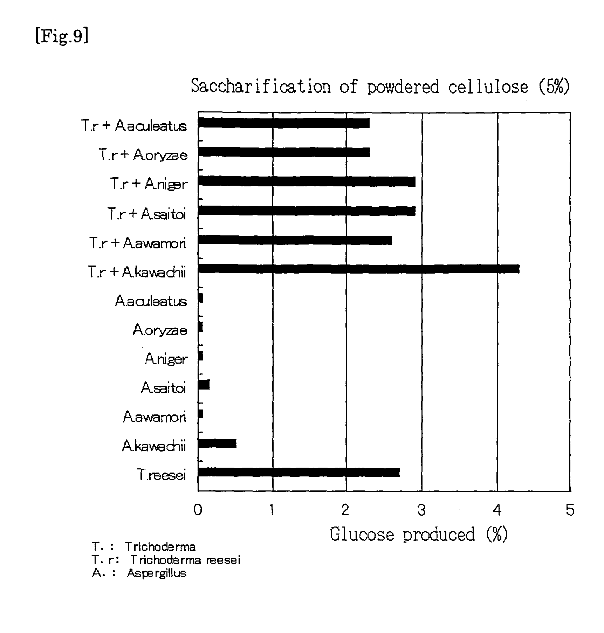

Saccharification was carried out by shaking a liquid (a 5% powdered cellulose liquid) consisting of powdered cellulose: 0.5 g, a mixture of the culture supernatant liquid of the genus Aspergillus filamentous fungi and the culture supernatant liquid of Trichoderma reesei (the mixing ratio is 50 : 50): 9.3 ml, and a 1 M acetate buffer (pH 4.8): 0.2 ml at 50°C at pH 4.8 for 24 hours. Then, glucose thus produced was measured. As a control, the culture supernatant liquid of Trichoderma reesei: 9.3 ml and the culture supernatant liquid of the genus Aspergillus: 9.3 ml were each singly used instead of the mixture of the culture supernatant liquids. The results thus obtained are shown in Fig. 9.

### Reference Example 1

The synergistic effects of Trichoderma-derived cellulase and Aspergillus kawachii-derived cellulase were examined. A Trichoderma reesei-derived cellulase preparation (Sigma-Aldrich, Inc.) and a Trichoderma viride-derived cellulase preparation (Sigma-Aldrich, Inc.) were each dissolved in water to prepare 1% cellulase liquids. Saccharification was carried out by shaking a liquid (a 5% bagasse liquid) consisting of delignified bagasse: 0.5 g, a 1% cellulase liquid: 4.65 ml, a culture supernatant liquid of Aspergillus kawachii: 4.65 ml and a 1 M acetate buffer (pH 4.8): 0.2 ml at 50°C at pH 4.8 for 24 hours. Then, glucose thus produced was measured. For the culture supernatant liquid of Aspergillus kawachii, one prepared in a similar manner to Example 1 was used.

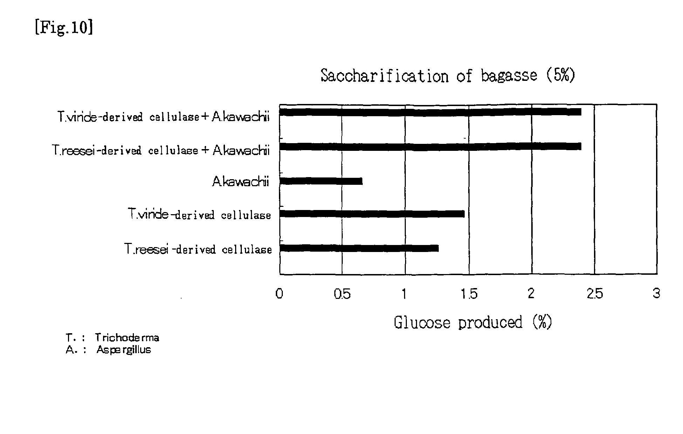

As a control, the 1% cellulase liquid: 9.3 ml and the culture supernatant liquid of Aspergillus kawachii: 9.3 ml were each singly used instead of the mixture of the culture supernatant liquids. The results thus obtained are shown in Fig. 10.

From the results of the examples and reference example obtained as described above, when a culture liquid or a supernatant liquid of the genus Trichoderma filamentous fungus is used in combination with a culture liquid or a supernatant liquid of Aspergillus kawachii, synergistic effect was identified in cellulolytic activity, and cellulose degradation and saccharification ability of the culture liquid or the supernatant liquid of the filamentous fungus was remarkably enhanced.

### Example 6

A culture supernatant liquid of Trichoderma reesei was obtained in a similar manner to Example 1.

Aspergillus kawachii (NBRC4308) was cultured on a potato dextrose agar medium at 37°C for 4 days so as to allow spores to be fully formed. One platinum loop thereof was inoculated into a medium obtained by autoclaving a petri dish containing 50 g of bran adjusted to have a water content of 50% at 121°C for 15 minutes. The spores were then cultured at 36°C for 3 days to give a solid culture product. To the solid culture product, 0.5% brine was added in an amount five times the weight of the solid culture product. After letting the solid culture product stand overnight, it was filtrated to remove the fungus body, whereby a solid culture filtrate liquid was obtained.

The hemicellulase activity of the solid culture filtrate liquid thus obtained was 90 U/ml as measured by the above-described method.

The cellulose raw material to be saccharified was subjected to delignification in accordance with the following method. Pieces of bagasse were each finely pulverized and suspended in 0.3 N NaOH, and treated at 120°C for 15 minutes. Subsequently, the product thus obtained was thoroughly washed with water and dried. Powdered cellulose (manufactured by Nippon Paper Chemicals Co., Ltd., trade name: KC flock W-50) was subjected to saccharification as it was.

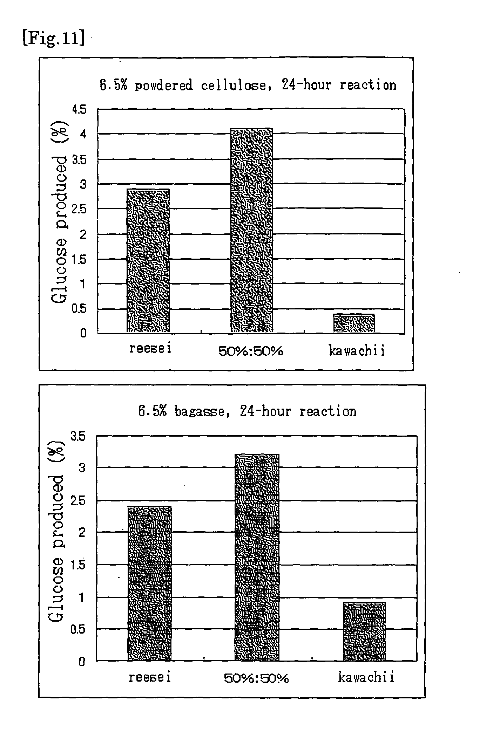

The cellulose raw material was saccharified by shaking a liquid (a 6.5% cellulose raw material liquid) consisting of the cellulose raw material: 0.65 g, the culture supernatant liquid of Trichoderma reesei: 4.6 ml, the solid culture filtrate liquid of Aspergillus kawachii: 4.6 ml, and a 1 M acetate buffer (pH 4.8): 0.2 ml (a volume ratio of the culture supernatant liquid to the solid culture filtrate liquid was 50% : 50%) at 50°C at pH 4.8 for 24 hours. Then, glucose thus produced was measured by glucose CII-test Wako (Wako Pure Chemicals Industries, Ltd.). As a control, the culture supernatant liquid of Trichoderma reesei: 9.2 ml and the solid culture filtrate liquid of Aspergillus kawachii: 9.2 ml were each singly used instead of the mixture of the above. The results thus obtained are shown in Fig. 11. The mixing ratio shown in the horizontal axis of the graph is the volume ratio.

### BRIEF DESCRIPTION OF THE DRAWINGS

[Fig. 1] Fig. 1 is a graph in which the concentration of glucose produced is plotted with respect to the reaction time for saccharification of beer residue.
[Fig. 2] Fig. 2 is a graph in which the concentration of glucose produced is plotted with respect to the reaction time for saccharification of rice straw.
[Fig. 3] Fig. 3 is a graph in which the concentration of glucose produced is plotted with respect to the reaction time for saccharification of wheat straw.
[Fig. 4] Fig. 4 is a graph in which the concentration of glucose produced is plotted with respect to the reaction time for saccharification of bagasse.
[Fig. 5] Fig. 5 is a graph in which the concentration of glucose produced is plotted with respect to the reaction time for saccharification of powdered cellulose.
[Fig. 6] Fig. 6 is a graph comparing the concentrations of glucose produced when bagasse was saccharified with altering the mixing ratio of the culture supernatant liquid of Trichoderma reesei and the culture supernatant liquid of Aspergillus kawachii.
[Fig. 7] Fig. 7 is a graph comparing the concentrations of glucose produced when powdered cellulose was saccharified with altering the mixing ratio of the culture liquid of Trichoderma reesei and the culture liquid of Aspergillus kawachii.
[Fig. 8] Fig. 8 is a graph comparing the concentrations of glucose produced when beer residue was saccharified with altering the mixing ratio of the culture supernatant liquid of Trichoderma reesei and the culture supernatant liquid of Aspergillus kawachii.
[Fig. 9] Fig. 9 is a graph comparing the concentrations of glucose produced when powdered cellulose was saccharified by using various kinds of the culture supernatant liquids of the genus Aspergillus filamentous fungi in combination with the culture supernatant liquid of Trichoderma reesei.
[Fig. 10] Fig. 10 is a graph comparing the concentrations of glucose produced when powdered cellulose was saccharified by using liquids containing cellulase derived from two kinds of the genus Trichoderma filamentous fungi in combination with the culture supernatant liquid of Aspergillus kawachii.
[Fig. 11] Fig. 11 is a graph showing the concentrations of glucose produced when cellulose raw materials were saccharified with using the culture supernatant liquid of Trichoderma reesei in combination with the solid culture filtrate liquid of Aspergillus kawachii.

## Claims

1. A mixture of a culture liquid or a supernatant liquid of a genus Trichoderma filamentous fungus and a culture product of Aspergillus kawachii, or a dried product thereof, which exhibits cellulolytic activity.

2. The mixture or the dried product thereof according to claim 1, wherein the culture product of Aspergillus kawachii is a culture liquid or a supernatant liquid.

3. The mixture or the dried product thereof according to claim 1, wherein the culture product of Aspergillus kawachii is a solid culture product or an extract liquid thereof.

4. The mixture or the dried product thereof according to any one of claims 1 to 3, wherein cellulase activity of the culture liquid or the supernatant liquid of the genus Trichoderma filamentous fungus is not less than 50 U/ml, and hemicellulase activity of the culture liquid, the supernatant liquid or the solid culture product extract liquid liquid of Aspergillus kawachii is not less than 50 U/ml.

5. The mixture or the dried product thereof according to any one of claims 1 to 4, wherein mixing ratio of the culture liquid or the supernatant liquid of the genus Trichoderma filamentous fungus to the culture liquid, the supernatant liquid, or the solid culture product extract liquid liquid of Aspergillus kawachii is within a range of 10 : 90 to 90 : 10 in volume ratio.

6. The mixture or the dried product thereof according to any one of claims 1 to 5, wherein the genus Trichoderma filamentous fungus is Trichoderma reesei or Trichoderma viride.

7. The mixture or the dried product thereof according to any one of claims 1 to 5, wherein the genus Trichoderma filamentous fungus is Trichoderma reesei.

8. A method for producing glucose comprising a step of saccharifying a cellulose raw material using the mixture or the dried product thereof according to any one of claims 1 to 7.

9. The method according to claim 8, wherein the saccharification is performed under conditions of 30 to 70°C and pH 3 to 7.
